# FASCICULE DE BREVET EUROPEEN

(11) **EP 1 917 988 B1**
(45) Date de publication et mention de la délivrance du brevet: **09.02.2011**
(21) Numéro de dépôt: 07291235.5
(22) Date de dépôt: 10.10.2007
(51) Int. Cl.: A61M 1/02, A61M 39/18, A61L 2/00, A61J 1/14, A61J 1/20

(54) **Récipient équipé d'un système de transfert aseptique**
Behälter, der mit einem aseptischen Transfersystem ausgestattet ist
Container equipped with an aseptic transfer system

(30) Priorité: 31.10.2006 FR 0609571
(43) Date de publication de la demande: 07.05.2008
(73) Titulaire: Maco Pharma, 59400 Mouvaux (FR)
(72) Inventeur: Goudaliez, Francis, 59155 Faches-Thumesnil (FR); Verpoort, Thierry, 59250 Halluin (FR)
(74) Mandataire: Sayettat, Julien Christian

(56) Documents cités:
- EP-A- 0 225 861
- FR-A1- 2 773 735
- US-A- 5 289 858

## Description

L'invention concerne un récipient destiné à contenir un fluide, ledit récipient étant équipé d'un système de transfert de façon stérile du fluide à l'intérieur dudit récipient. L'invention concerne également un procédé de transfert aseptique d'un fluide depuis un premier récipient dans un récipient selon l'invention.

Elle s'applique typiquement au cas où le premier récipient est une poche souple contenant un fluide médical et le récipient selon l'invention est un flacon rigide.

Dans le domaine de la transfusion sanguine, il est classique de prélever le sang et séparer les composants sanguins tels que le plasma et les concentrés de globules rouges dans des systèmes à poches tels que ceux décrits dans le document EP-A1-526678. Ces systèmes, dits clos, permettent de garantir la stérilité des composants sanguins séparés.

Une fois séparés dans différentes poches, les composants sanguins peuvent subir différents traitements tels que la filtration, la décontamination bactérienne et/ou virale ou la lyophilisation.

Or, certains traitements ne peuvent pas être réalisés dans les poches du système clos utilisé pour le prélèvement ou la séparation des composants sanguins. Par exemple, certains traitements nécessitent de transférer les composants sanguins dans des flacons rigides, notamment en verre.

Il existe de nombreux types de dispositifs de transferts dans le domaine médical, notamment pour réaliser la reconstitution médicamenteuse d'un médicament contenu dans un flacon en verre vers une poche de perfusion. Un tel dispositif est décrit par exemple dans le document EP-A1-1034772. Toutefois, ce type de dispositifs ne permet pas le transfert aseptique de fluide entre une poche et un flacon.

Un autre type de dispositif de transfert entre un tube et un autre récipient est décrit dans le document EP-A1-0225861. Dans ce document, le tube est disposé dans une poche ouverte sur le bas et fermable. La poche est munie d'un col au travers duquel une tubulure s'étend. L'extrémité de la tubulure est pourvue d'un connecteur pour connecter l'autre récipient. Ce dispositif prévu pour éviter de contaminer le manipulateur ne permet pas de transfert aseptique.

Le document EP-A1-0548577 décrit un bouchon pour flacon comprenant une tubulure fermée destinée à être connectée de façon stérile à une autre tubulure fermée, par exemple celle d'une poche pour perfusion ou d'une seringue. Ce bouchon particulier permet de réaliser le transfert en système clos du fluide contenu dans le flacon vers la poche ou la seringue.

Cette réalisation nécessite la réalisation d'un bouchon particulier ce qui, outre l'impact économique, contraint les manipulations ultérieures du flacon bouché. En outre, la fonction du bouchon peut être dégradée par la réalisation du passage de la tubulure au travers de lui.

L'invention vise à résoudre les problèmes de l'art antérieur en fournissant notamment un récipient équipé d'un système pour le transfert stérile qui ne nécessite pas la fabrication d'un bouchon particulier et qui permet d'utiliser classiquement le récipient après le transfert.

A cet effet, et selon un premier aspect, l'invention propose un récipient comprenant un corps destiné à contenir un fluide et un orifice par l'intermédiaire duquel ledit fluide peut être introduit dans ledit corps, ledit récipient étant équipé d'un système de transfert de façon stérile du fluide à l'intérieur dudit corps, ledit récipient étant caractérisé en ce que ledit système de transfert comprend :
- une paroi déformable et étanche, ladite paroi étant disposée par rapport au récipient de sorte à former un espace clos et stérile dans lequel l'orifice est disposé ;
- un dispositif de communication fluidique entre l'espace clos et l'extérieur du récipient, ledit dispositif étant actionnable de façon stérile ;
- un moyen de bouchage de l'orifice, ledit moyen étant au moins partiellement disposé dans l'espace clos de sorte à être actionnable entre une position d'ouverture et une position de fermeture de l'orifice.

Selon un deuxième aspect, l'invention concerne un procédé de transfert aseptique d'un fluide depuis un premier récipient dans un récipient selon le premier aspect, ledit procédé comprenant les étapes prévoyant de :
- mettre en communication les deux récipients par actionnement de façon stérile du dispositif de communication fluidique ;
- transférer le fluide depuis le premier récipient dans le corps du deuxième récipient ;
- actionner le moyen de bouchage depuis sa position d'ouverture vers sa position de fermeture par déformation de la paroi.

D'autres objets et avantages apparaîtront au cours de la description qui suit en référence aux dessins annexés.

Les figures 1 et 6 représentent une vue schématique de face d'un récipient selon deux modes différents de réalisation de l'invention.

Les figures 2 à 5 représentent de façon schématique les étapes du procédé selon l'invention de transfert aseptique depuis une poche vers le récipient de la figure 1.

La figure 7 représente une variante du mode de réalisation du récipient de la figure 6.

Dans la description qui suit, les termes amont et aval sont définis par rapport au sens d'écoulement du fluide lors du transfert aseptique, c'est-à-dire du premier récipient vers un récipient selon l'invention.

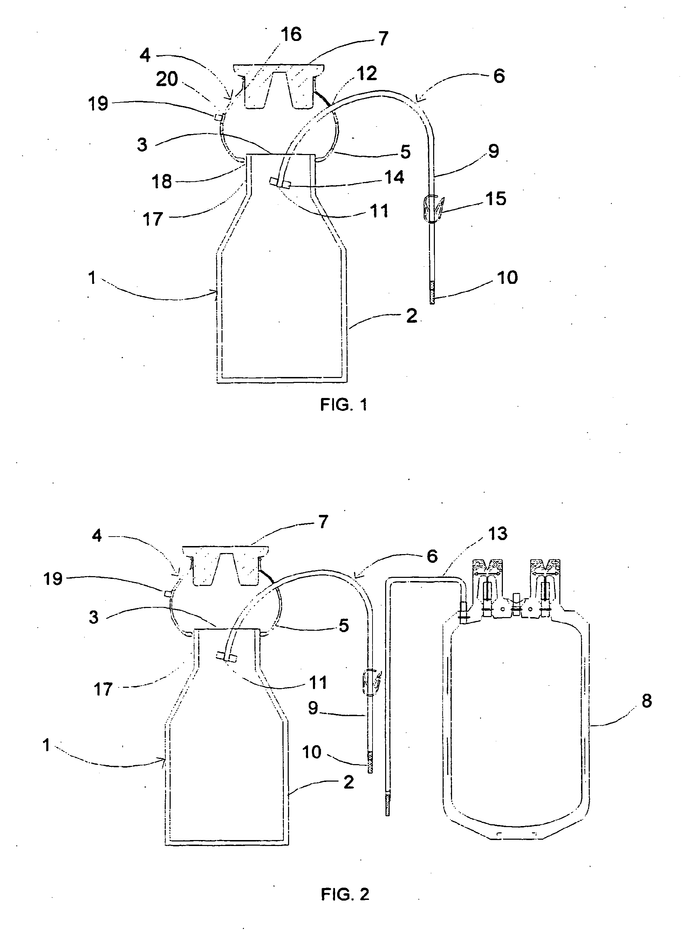

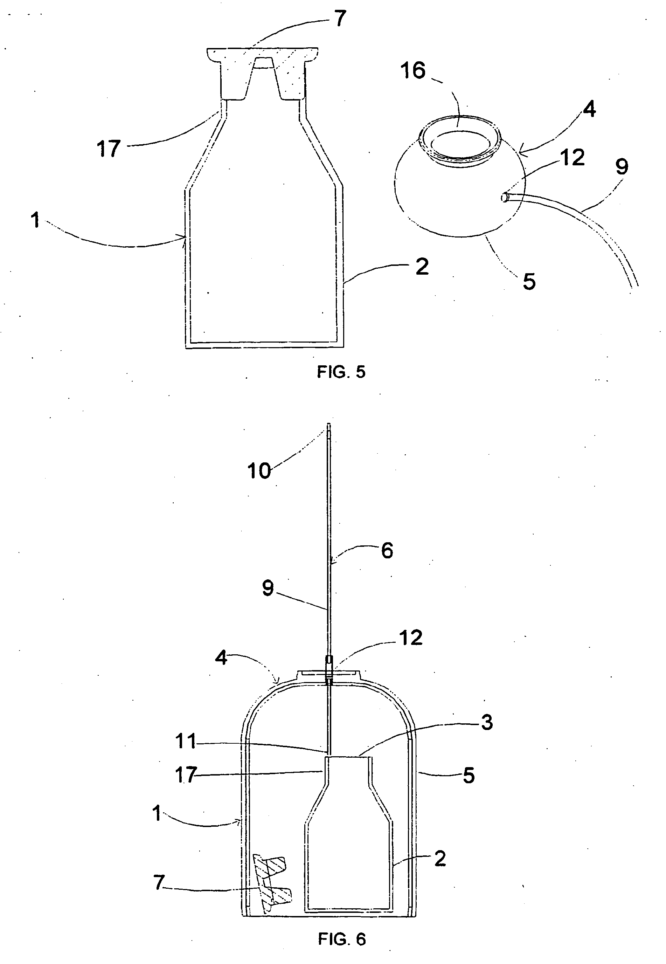

Selon la figure 1 ou 6, le récipient 1 selon le premier aspect de l'invention comprend un corps 2 destiné à contenir un fluide et un orifice 3 par l'intermédiaire duquel ledit fluide peut être introduit dans ledit corps 2.

Le récipient est réalisé dans un matériau souple ou rigide, stérilisable notamment par vapeur, oxyde d'éthylène, irradiation gamma ou irradiation bêta. En particulier, le récipient est un flacon ou une bouteille en verre.

Le récipient 1 est équipé d'un système 4 de transfert de façon stérile du fluide à l'intérieur dudit corps 2, ledit système de transfert comprenant :
- une paroi 5 déformable et étanche, ladite paroi étant disposée par rapport au récipient 1 de sorte à former un espace clos et stérile dans lequel l'orifice 3 est disposé ;
- un dispositif 6 de communication fluidique entre l'espace clos et l'extérieur du récipient, ledit dispositif étant actionnable de façon stérile ;
- un moyen 7 de bouchage de l'orifice, ledit moyen 7 étant au moins partiellement disposé dans l'espace clos de sorte à être actionnable entre une position d'ouverture et une position de fermeture de l'orifice 3.

Ce système 4 de transfert permet le transfert stérile d'un fluide contenu dans un premier récipient vers le récipient 1, c'est-à-dire le transfert du fluide sans mettre en contact le fluide avec l'air ambiant et les contaminants éventuellement présents dans l'air ambiant.

Le système 4 comprend une paroi 5 déformable et étanche formant un espace clos et stérile dans lequel l'orifice 3 du récipient est disposé. Ainsi, l'intérieur du récipient est également stérile.

La stérilité de l'espace clos est obtenue lors de la fabrication du récipient, par stérilisation du récipient équipé du système de transfert. La méthode de stérilisation dépend des matériaux utilisés pour la fabrication des différents composants du récipient.

La paroi 5 du système de transfert 4 est déformable de sorte à pouvoir actionner de l'extérieur et manuellement les composants contenus dans l'espace clos, et notamment le moyen 7 de bouchage de l'orifice.

La paroi 5 est étanche pour préserver la stérilité du récipient et de l'espace clos formé par la paroi.

Selon une réalisation particulière, la paroi est souple et réalisée en matériau polymère, notamment, en un matériau stérilisable par l'une des méthodes énoncées ci-dessus, par exemple en polychlorure de vinyle ou en silicone. Le choix du matériau dépend de sa tenue à la stérilisation et de ses propriétés mécaniques.

Sur la figure 1 ou 5, la paroi 5 prend la forme d'un manchon souple, dont l'une des extrémités est assemblée autour de l'orifice 3 du récipient et l'autre extrémité autour du moyen de bouchage 7. Le manchon est par exemple moulé par injection.

Sur la figure 6, la paroi 5 est formée de deux feuilles souples assemblées sur leur périphérie de sorte à former une enveloppe.

Selon une autre réalisation (non représentée), la paroi 5 est rigide ou semi rigide et comprend des soufflets ou plis rendant la paroi flexible.

Le système comprend en outre un dispositif 6 de communication fluidique entre l'espace clos et l'extérieur du récipient. Ce dispositif 6 de communication est actionnable de façon stérile, c'est-à-dire que lorsqu'il est actionné, il ne détruit pas la stérilité du récipient.

En relation avec la figure 2, le dispositif de communication fluidique 6 est destiné à permettre le transfert d'un fluide contenu dans un premier récipient 8 placé à l'extérieur du récipient 1 selon l'invention vers l'intérieur dudit récipient 1.

Ce transfert est réalisé de façon aseptique, de sorte à ne pas contaminer le fluide avec des contaminants de l'air ambiant.

Selon une réalisation particulière représentée sur les figures 1 ou 6, le dispositif de communication fluidique 6 comprend une tubulure souple 9 traversant la paroi 5, l'extrémité amont 10 de ladite tubulure étant disposée à l'extérieur de l'espace clos et l'extrémité aval 11 étant disposée à l'intérieur dudit espace.

A cet effet, la paroi 5 comprend un premier orifice 12 dont le diamètre est sensiblement égal au diamètre de la tubulure souple 9 de sorte à garantir l'étanchéité de la paroi 5. Pour améliorer cette étanchéité, l'orifice 12 est muni par exemple d'une bague en élastomère.

Selon les figures 1 à 6, l'extrémité amont 10 de la tubulure est fermée et l'extrémité aval 11 de la tubulure est ouverte.

La tubulure 9 est réalisée dans un matériau sécable et soudable, tel que le polychlorure de vinyle. Ainsi, il est possible de réaliser une connexion stérile avec une autre tubulure 13 fermée prévue sur un premier récipient 8 à l'aide d'un appareil de connexion stérile. Un tel appareil est disponible dans le commerce et comprend le SCD® 312 fabriqué par Terumo.

Le procédé de connexion stérile mis en oeuvre par cet appareil est décrit par exemple, dans les documents EP-044 204, EP-0 134 630 et EP-0 208 004.

Dans une variante non représentée, l'extrémité amont 10 de la tubulure 9 est munie d'un raccord qui peut être connecté de façon stérile à un autre raccord relié à un premier récipient 8 contenant un fluide. De tels raccords sont par exemple décrits dans le document EP-0 756 121.

Dans ces deux variantes, la connexion entre le récipient 1 de l'invention et le premier récipient 8 contenant le fluide doit être stérile, c'est-à-dire sans que le fluide puisse entrer en contact avec les contaminants de l'air ambiant.

Ce récipient permet donc de connecter stérilement un premier récipient 8 contenant un fluide, telle qu'une poche, à un récipient 1 tel qu'un flacon ou une bouteille en verre. Ce récipient équipé d'un système de transfert 4 évite la pré-connexion du premier récipient 8 au moment de la fabrication. Il n'est pas non plus nécessaire de recourir à une stérilisation ultérieure de la connexion entre les deux récipients 1,8 ou du fluide.

Pour facilité la manipulation du récipient, la tubulure 9 est montée coulissante et de façon étanche au travers de la paroi 5. Ainsi, la tubulure 9 peut être initialement introduite dans le corps 2 du récipient 1, et après le transfert du fluide, retirée par traction pour permettre la mise en place du moyen de bouchage 7.

Pour éviter de rompre la stérilité de l'espace clos en faisant pénétrer dans l'espace clos une partie de la tubulure initialement à l'extérieur du récipient, et donc potentiellement contaminée, la tubulure 9 coulisse vers l'extérieur de l'espace clos et est empêcher de coulisser vers l'intérieur.

Pour cela, la tubulure 9 est par exemple, munie de moyens de blocage, tels que des rainures.

La partie de la tubulure disposée dans l'espace clos est munie d'un moyen formant butée 14 lors du coulissement de la tubulure au travers de la paroi.

Selon la figure, le moyen formant butée est un arrêtoir disposé à l'extrémité aval 11 de la tubulure, empêchant le retrait total de la tubulure 9 de l'espace clos. Ainsi, la stérilité de l'espace clos est préservé.

Pour contrôler l'écoulement du fluide dans la tubulure, celle-ci est munie d'un moyen de contrôle 15 de l'écoulement dans la tubulure tel qu'un clamp ou un ouvre-circuit.

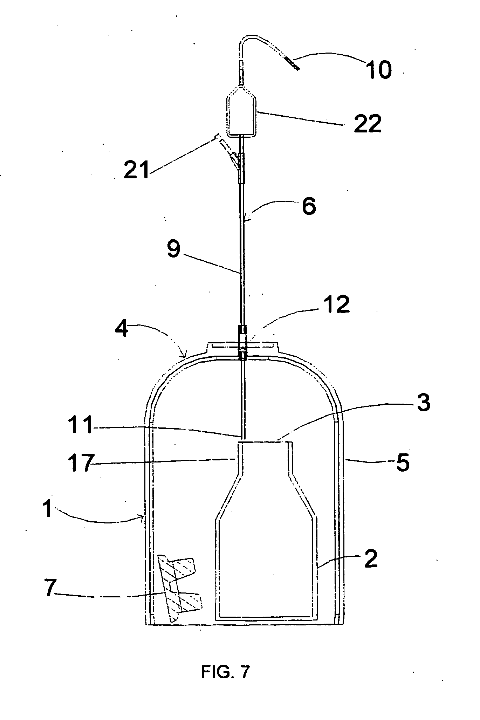

Selon une variante représentée sur la figure 7, la tubulure souple comprend un moyen d'aération 21 tel qu'une prise d'air ou un évent. Le moyen d'aération 21 est capable de laisser passer les gaz. Le moyen d'aération 21 est par exemple arrangé sur une tubulure branchée sur la tubulure 9 traversant la paroi du système de transfert 4.

Le moyen d'aération 21 est choisi de sorte à ne pas compromettre la stérilité récipient 1. Par exemple, la prise d'air ou l'évent comprend une membrane ayant une taille de pore suffisamment petite pour empêcher le passage des bactéries dans ledit récipient. La taille de pores est notamment inférieure ou égale à environ 0,22 µm.

Ce moyen d'aération 21 est utile lors de la fabrication du récipient et particulièrement lors de la stérilisation du récipient 1 équipé du système de transfert.

En effet, lors de la stérilisation par vapeur, le système de transfert se gonfle par l'effet de la dilatation de l'air contenu dans le système. Cette surpression entraîne un risque d'éclatement du système de transfert 4. Le moyen d'aération 21 permet d'éviter cette surpression en permettant à une partie de l'air d'être purgé du système.

Selon une autre variante représentée sur la figure 7, la tubulure 9 est munie d'un moyen de filtration du fluide destiné à être contenu dans le récipient 1. Le moyen de filtration prend notamment la forme d'un dispositif de filtration 22 apte à éliminer les pathogènes du fluide.

Dans le cas où le fluide est du plasma, le dispositif de filtration comprend par exemple une membrane ayant une taille de pore inférieure à 0,22 µm de sorte à éliminer les éventuelles bactéries contenues dans le plasma.

Ce dispositif de filtration garantit que le fluide transféré dans le récipient ne contient pas de pathogènes et notamment pas de bactéries.

Selon une autre variante non représentée, le moyen de filtration 22 joue également le rôle de moyen d'aération. Dans ce cas, l'extrémité aval 11 de la tubulure n'est pas fermée. Le moyen de filtration / aération est capable de laisser passer le gaz et les fluides.

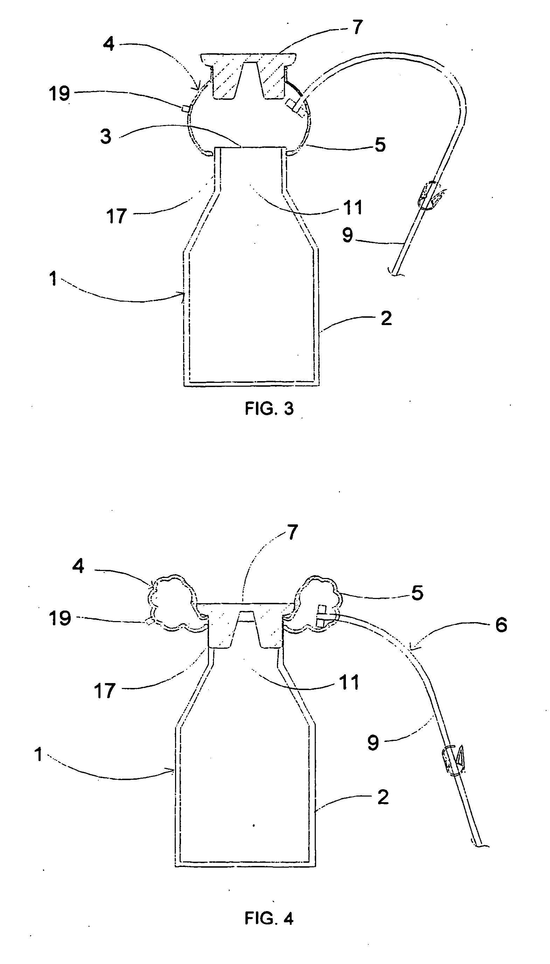

Le système de transfert 4 comprend enfin un moyen de bouchage 7 actionnable entre une position d'ouverture (figure 1) et une position de fermeture (figure 4) de l'orifice du récipient 1.

Selon une réalisation, le moyen de bouchage 7 prend la forme d'un bouchon qui peut être placé par enfoncement dans l'orifice 3 du récipient 1 pour le fermer.

En variante, le moyen de bouchage 7 peut être vissé ou clipsé sur l'orifice 3.

Selon la figure 1, le moyen de bouchage 7 est disposé au travers de la paroi 5 et en regard de l'orifice 3, ledit moyen de bouchage étant monté de façon étanche sur la paroi 5.

Pour cela, la paroi 5 comprend un deuxième orifice 16 dans lequel est disposé le moyen de bouchage 7. Comme le premier orifice 12, le deuxième orifice 16 peut comprendre une bague d'étanchéité.

Notamment, le moyen de bouchage 7 comprend un corps dont la partie inférieure est agencée pour s'engager dans l'orifice en vue de sa fermeture de façon étanche, ladite partie inférieure étant disposée dans l'espace clos.

Selon la figure 1, la partie supérieure du corps du moyen de bouchage 7 est disposée à l'extérieur de l'espace clos.

Ainsi, pour boucher le récipient 1 de l'invention, il suffit d'appuyer sur la partie supérieure du corps du moyen de bouchage 7, pour enfoncer la partie inférieure dans l'orifice 3 du récipient. La paroi déformable permet cette opération.

En relation avec les figures 1 à 5 et selon une première variante, l'orifice 3 du récipient est prévu sur un col 17, la paroi 5 étant disposée de façon étanche autour dudit col 17.

A cet effet, la paroi 5 est munie d'un troisième orifice 18 dans lequel est disposé le col 17 du récipient 1. Avantageusement, cet orifice 18 est muni d'une bague d'étanchéité.

Selon une autre variante représentée sur la figure 6, la paroi est disposée autour de l'ensemble du récipient 1 de sorte que ledit récipient soit contenu dans l'espace clos.

Pour faciliter la fabrication de la paroi 5, le moyen de bouchage 7 est contenu dans l'espace clos, seule la tubulure 9 formant dispositif de communication fluidique 6 traversant la paroi 5.

En particulier, la paroi 5 prend la forme d'un gant muni d'au moins deux doigts facilitant la manipulation du récipient 1 et du moyen de bouchage 7 de l'extérieur du récipient.

Avantageusement, la paroi est munie d'une prise d'air 19. La prise d'air 19 est placée dans un quatrième orifice 20 de la paroi 5. Cette prise d'air 19 permet le remplissage du récipient 1, notamment rigide, en chassant l'air de l'espace clos vers l'extérieur du récipient 1.

Selon un deuxième aspect, l'invention concerne un procédé de transfert aseptique d'un fluide depuis un premier récipient 8 dans un récipient 1 selon le premier aspect de l'invention, ledit procédé comprenant les étapes prévoyant de:
- mettre en communication les deux récipients 1,8 par actionnement de façon stérile du dispositif de communication fluidique 6 ;
- transférer le fluide depuis le premier récipient 8 dans le corps du récipient 1 selon l'invention ;
- actionner le moyen de bouchage 7 depuis sa position d'ouverture vers sa position de fermeture par déformation de la paroi 5.

Ce procédé s'applique particulièrement dans le cas d'un transfert de fluide stérile contenu dans un premier récipient 8 tel qu'une poche souple, vers un récipient rigide 1 tel qu'un flacon ou une bouteille en verre.

Selon les figures 1 à 7, lorsque le récipient 1 est équipé d'une tubulure 9, la mise en communication est réalisée par connexion stérile entre la tubulure 9 et une tubulure 13 fermée, soudable et sécable prévue sur le premier récipient 8 pour être en communication avec le fluide contenu dans ledit récipient 8.

Dans le cas du récipient de la figure 1, avant ou après la connexion stérile, l'extrémité aval 11 de la tubulure 9 est disposée à l'intérieur du corps 2 du récipient 1 pour permettre l'introduction du fluide dans ledit récipient (figure 2).

Après le transfert du fluide, la tubulure 9 est retirée de l'intérieur du corps 2 du récipient par traction sur celle-ci, tout en gardant son extrémité aval 11 à l'intérieur de l'espace clos (figure 3). Cette opération est sécurisée par la présence d'un arrêtoir 14 sur l'extrémité aval 11 de la tubulure 9.

Selon la figure 4, pour refermer le corps du récipient, on exerce une pression sur le moyen de bouchage 7 de sorte à l'introduire dans le col du récipient.

Une fois le récipient bouché, l'intérieur du corps du récipient 1 est clos et stérile. Si la paroi 5 est suffisamment souple et élastique, elle peut être retirée du col 17 du récipient, laissant le récipient bouché à l'air ambiant (figure 5). L'intérieur du récipient n'a jamais été mis en contact avec les contaminants de l'air ambiant, le transfert est aseptique.

Selon la figure 6, lorsque le récipient 1 et le moyen de bouchage 7 sont contenus dans l'espace clos, le procédé de transfert est réalisé comme suit.

Avant ou après la connexion stérile, le moyen de bouchage 7 est retiré du col du flacon 7 par manipulation de l'extérieur de la paroi 5.

La tubulure 9 est introduite dans le col du récipient 1 et l'on procède au transfert du fluide. A la fin du transfert, le moyen de bouchage 7 est placé sur le col du flacon pour le boucher.

La paroi 5 est ensuite ouverte, par exemple par découpage, pour récupérer le récipient bouché contenant le fluide.

Le récipient bouché contenant le fluide est ensuite utilisé de façon conventionnelle pour subir des traitements ultérieurs, comme par exemple la lyophilisation du fluide contenu à l'intérieur.

## Revendications

1. Récipient (1) comprenant un corps (2) destiné à contenir un fluide et un orifice (3) par l'intermédiaire duquel ledit fluide peut être introduit dans ledit corps, ledit récipient (1) étant équipé d'un système de transfert (4) de façon stérile du fluide à l'intérieur dudit corps (2), ledit récipient étant **caractérisé en ce que** ledit système de transfert comprend :
- une paroi (5) déformable et étanche, ladite paroi (5) étant disposée par rapport au récipient (1) de sorte à former un espace clos et stérile dans lequel l'orifice (3) est disposé ;
- un dispositif de communication fluidique (6) entre l'espace clos et l'extérieur du récipient, ledit dispositif (6) étant actionnable de façon stérile ;
- un moyen de bouchage (7) de l'orifice (3), ledit moyen étant au moins partiellement disposé dans l'espace clos de sorte à être actionnable entre une position d'ouverture et une position de fermeture de l'orifice.

2. Récipient selon la revendication 1, **caractérisé en ce que** la paroi (5) est réalisée en matériau polymère.

3. Récipient selon la revendication 1 ou 2, **caractérisé en ce qu'**il est réalisé dans un matériau rigide.

4. Récipient selon l'une quelconque des revendications 1 à 3, **caractérisé en ce que** le dispositif de communication fluidique (6) comprend une tubulure souple (9) traversant la paroi (5), l'extrémité amont (10) de ladite tubulure étant disposée à l'extérieur de l'espace clos et l'extrémité aval (11) étant disposée à l'intérieur dudit espace.

5. Récipient selon la revendication 4 **caractérisé en ce que** la tubulure souple (9) comprend un moyen d'aération (21) du système de transfert (4).

6. Récipient selon la revendication 4 ou 5 **caractérisé en ce que** la tubulure souple (9) est munie d'un moyen de filtration (22) des pathogènes du fluide destiné à être contenu dans le récipient (1).

7. Récipient selon l'une quelconque des revendications 4 à 6, **caractérisé en ce que** l'extrémité amont (10) de la tubulure (9) est fermée et l'extrémité aval (11) de la tubulure (9) est ouverte.

8. Récipient selon l'une quelconque des revendications 4 à 7, **caractérisé en ce que** la tubulure (9) est réalisée dans un matériau sécable et soudable.

9. Récipient selon l'une quelconque des revendications 4 à 8, **caractérisé en ce que** la tubulure (9) est montée coulissante et de façon étanche au travers de la paroi (5).

10. Récipient selon la revendication 9, **caractérisé en ce que** la partie de la tubulure disposée dans l'espace clos est munie d'un moyen formant butée (14) lors du coulissement de la tubulure (9) au travers de la paroi (5).

11. Récipient selon l'une quelconque des revendications 1 à 10, **caractérisé en ce que** le moyen de bouchage (7) est disposé au travers de la paroi (5) et en regard de l'orifice (3), ledit moyen de bouchage (7) étant monté de façon étanche sur la paroi (5).

12. Récipient selon la revendication 11, **caractérisé en ce que** le moyen de bouchage (7) comprend un corps dont la partie inférieure est agencée pour s'engager dans l'orifice (3) en vue de sa fermeture de façon étanche, ladite partie inférieure étant disposée dans l'espace clos.

13. Récipient selon l'une quelconque des revendications 1 à 12, **caractérisé en ce que** l'orifice (3) est prévu sur un col (17), la paroi (5) étant disposée de façon étanche autour dudit col (17).

14. Récipient selon l'une quelconque des revendications 1 à 13, **caractérisé en ce que** la paroi (5) est disposée autour de l'ensemble du récipient (1) de sorte que ledit récipient soit contenu dans l'espace clos.

15. Récipient selon l'une quelconque des revendications 1 à 14, **caractérisé en ce que** la paroi est munie d'une prise d'air (19).

16. Procédé de transfert aseptique d'un fluide depuis un premier récipient (8) dans un récipient selon l'une quelconque des revendications 1 à 15, ledit procédé comprenant les étapes prévoyant de :
- mettre en communication les deux récipients (1,8) par actionnement de façon stérile du dispositif de communication fluidique (6) ;
- transférer le fluide depuis le premier récipient (8) dans le corps (2) du deuxième récipient ;
- actionner le moyen de bouchage (7) depuis sa position d'ouverture vers sa position de fermeture par déformation de la paroi (5).

17. Procédé de transfert selon la revendication 16 lorsqu'elle dépend de la revendication 4, **caractérisé en ce que** la mise en communication est réalisée par connexion stérile entre la tubulure (9) et une tubulure (13) soudable et sécable prévue sur le premier récipient (8) pour être en communication avec le fluide contenu dans ledit récipient (8).

## Claims

1. Vessel (1) comprising a body (2) intended to contain a fluid and an orifice (3) via which said fluid can be introduced into said body, said vessel (1) being equipped with a system (4) for transferring the fluid sterilely inside said body (2), said vessel being **characterised in that** said transfer system comprising:
- a deformable tight wall (5), said wall (5) being arranged in relation to the vessel (1) so as to form an sterile enclosed space in which the orifice (3) is arranged;
- a fluidic communication device (6) between the enclosed space and the outside of the vessel, said device (6) being suitable for sterile actuation;
- means (7) for sealing the orifice (3), said means being at least partially arranged in the enclosed space so as to be suitable for actuation between an open position and a closed position of the orifice.

2. Vessel according to claim 1, **characterised in that** the wall (5) is made of polymer material.

3. Vessel according to claim 1 or 2, **characterised in that** it is made of a rigid material.

4. Vessel according to any of claims 1 to 3, **characterised in that** the fluidic communication device (6) comprises a flexible tube (9) passing through the wall (5), the upstream end (10) of said tube being arranged outside the enclosed space and the downstream end (11) being arranged inside said space.

5. Vessel according to claim 4, **characterised in that** the flexible tube (9) comprises transfer system (4) aeration means (21).

6. Vessel according to claim 4 or 5 **characterised in that** the flexible tube (9) is provided with means (22) for filtering pathogens from the fluid to be contained in the vessel (1).

7. Vessel according to any of claims 4 to 6, **characterised in that** the upstream end (10) of the tube (9) is closed and the downstream end (11) of the tube (9) is open.

8. Vessel according to any of claims 4 to 7, **characterised in that** the tube (9) is made of a material suitable for breaking and welding.

9. Vessel according to any of claims 4 to 8, **characterised in that** the tube (9) is slidably mounted tightly through the wall (5).

10. Vessel according to claim 9, **characterised in that** the part of the tube arranged in the enclosed space is provided with means acting as an abutment (14) during the sliding of the tube (9) through the wall (5).

11. Vessel according to any of claims 1 to 10, **characterised in that** the sealing means (7) are arranged through the wall (5) and facing the orifice (3), said sealing means (7) being mounted tightly on the wall (5).

12. Vessel according to claim 11, **characterised in that** the sealing means (7) comprise a body wherein the lower part is arranged to be inserted in the orifice (3) with a view to closing same tightly, said lower part being arranged in the enclosed space.

13. Vessel according to any of claims 1 to 12, **characterised in that** the orifice (3) is provided on a neck (17), the wall (5) being arranged tightly about said neck (17).

14. Vessel according to any of claims 1 to 13, **characterised in that** the wall (5) is arranged about the entire vessel (1) such that the vessel is contained in the enclosed space.

15. Vessel according to any of claims 1 to 14, **characterised in that** the wall is provided with an air intake (19).

16. Method for the aseptic transfer of a fluid from a first vessel (8) into a vessel according to any of claims 1 to 15, said method comprising steps for:
- connecting the two vessel (1,8) by sterile actuation of the fluidic communication device (6);
- transferring the fluid from the first vessel (8) into the body (2) of the second vessel;
- actuating the sealing means (7) from the open position thereof to the closed position thereof by deforming the wall (5).

17. Transfer method according to claim 16 dependent on claim 4, **characterised in that** the connection is provided by a sterile connection between the tube (9) and a tube (13) suitable for welding and breaking provided on the first vessel (8) to be connected to the fluid contained in said vessel (8).

## Patentansprüche

1. Behälter (1), der einen Körper (2) enthält, der dazu bestimmt ist, ein Fluid einzuschließen, sowie eine Öffnung (3), durch die das besagte Fluid in den besagten Körper eingebracht werden kann, wobei der besagte Behälter (1) mit einem System zur keimfreien Überstellung (4) des Fluides ins Innere des besagten Körpers (2) ausgestattet ist, und der besagte Behälter **dadurch gekennzeichnet ist, dass** das besagte Überstellungssystem folgendes enthält:
- eine verformbare und dichte Wand (5), wobei die besagte Wand (5) so im Bezug zum Behälter (1) angeordnet ist, dass sie einen abgeschlossenen und keimfreien Raum bildet, in dem die besagte Öffnung (3) angeordnet ist;
- eine Vorrichtung zur Strömungsverbindung (6) zwischen dem abgeschlossenen Raum und der Außenseite des Behälters, wobei die besagte Vorrichtung (6) keimfrei betätigt werden kann;
- eine Vorrichtung zum Abdichten (7) der Öffnung (3), wobei die besagte Vorrichtung zumindest teilweise im abgeschlossenen Raum angeordnet ist, sodass sie zwischen einer offenen und einer geschlossenen Stellung der Öffnung betätigt werden kann.

2. Behälter nach Anspruch 1, **dadurch gekennzeichnet, dass** die Wand (5) aus einem Polymerwerkstoff gefertigt ist.

3. Behälter nach Anspruch 1 oder 2, **dadurch gekennzeichnet, dass** er aus einem steifen Material gefertigt ist.

4. Behälter nach irgendeinem der Ansprüche 1 bis 3, **dadurch gekennzeichnet, dass** die Vorrichtung zur Strömungsverbindung (6) einen biegsamen Rohrstutzen (9) enthält, der durch die Wand (5) führt, wobei das vorgelagerte Ende (10) des besagten Rohrstutzens außerhalb des abgeschlossenen Raumes, und das nachgelagerte Ende (11) innerhalb des besagten Raumes angeordnet sind.

5. Behälter nach Anspruch 4, **dadurch gekennzeichnet, dass** der biegsame Rohrstutzen (9) eine Vorrichtung zum Belüften (21) des Überstellungssystems (4) enthält.

6. Behälter nach Anspruch 4 oder 5, **dadurch gekennzeichnet, dass** der biegsame Rohrstutzen (9) mit einer Vorrichtung zum Filtern (22) der Krankheitserreger innerhalb des Fluides ausgestattet ist, das dazu bestimmt ist, im Behälter (1) eingeschlossen zu werden.

7. Behälter nach irgendeinem der Ansprüche 4 bis 6, **dadurch gekennzeichnet, dass** das vorgelagerte Ende (10) des Rohrstutzens (9) geschlossen, und das nachgelagerte Ende (11) des Rohrstutzens (9) offen ist.

8. Behälter nach irgendeinem der Ansprüche 4 bis 7, **dadurch gekennzeichnet, dass** der Rohrstutzen (9) aus einem teilbaren und schweißbaren Werkstoff gefertigt ist.

9. Behälter nach irgendeinem der Ansprüche 4 bis 8, **dadurch gekennzeichnet, dass** der Rohrstutzen (9) gleitend und dicht durch die Wand (5) geführt wird.

10. Behälter nach Anspruch 9, **dadurch gekennzeichnet, dass** der Abschnitt des Rohrstutzens, der im abgeschlossenen Raum angeordnet ist, mit einer Vorrichtung ausgestattet ist, die einen Anschlag (14) bildet, wenn der Rohrstutzen (9) durch die Wand (5) geschoben wird.

11. Behälter nach irgendeinem der Ansprüche 1 bis 10, **dadurch gekennzeichnet, dass** die Abdichtvorrichtung (7) durch die Wand (5) hindurch und gegenüber der Öffnung (3) angeordnet ist, wobei die besagte Abdichtvorrichtung (7) in dichter Form an der Wand (5) montiert ist.

12. Behälter nach Anspruch 11, **dadurch gekennzeichnet, dass** die Abdichtvorrichtung (7) einen Körper enthält, dessen unterer Abschnitt so angeordnet ist, dass er sich in die Öffnung (3) einfügt, um einen dichten Verschluss zu bilden, wobei der besagte untere Abschnitt im abgeschlossenen Raum angeordnet ist.

13. Behälter nach irgendeinem der Ansprüche 1 bis 12, **dadurch gekennzeichnet, dass** die Öffnung (3) an einem Hals (17) vorgesehen ist, und die Wand (5) in dichter Form um den besagten Hals (17) herum angeordnet ist.

14. Behälter nach irgendeinem der Ansprüche 1 bis 13, **dadurch gekennzeichnet, dass** die Wand (5) um die Behältereinheit (1) angeordnet ist, sodass der besagte Behälter im abgeschlossenen Raum enthalten ist.

15. Behälter nach irgendeinem der Ansprüche 1 bis 14, **dadurch gekennzeichnet, dass** die Wand mit einem Luftanschluss (19) ausgestattet ist.

16. Verfahren zur keimfreien Überstellung eines Fluides von einem ersten Behälter (8) in einen Behälter nach irgendeinem der Ansprüche 1 bis 15, wobei das besagte Verfahren Schritte enthält die folgendes vorsehen:
- die Verbindung zwischen den beiden Behältern (1, 8) herstellen, indem man die Strömungsverbindung (6) keimfrei betätigt;
- das Fluid vom ersten Behälter (8) in den Körper (2) des zweiten Behälters überstellen;
- die Abdichtvorrichtung (7) durch Verformen der Wand (5) von der offenen in die geschlossene Stellung bringen.

17. Überstellungsverfahren nach Anspruch 16, falls diese vom Anspruch 4 abhängig ist, **dadurch gekennzeichnet, dass** der Verbindungsaufbau durch die keimfreie Verbindung zwischen dem Rohrstutzen (9) und einem anschweißbaren und trennbaren Rohrstutzen (13) erfolgt, der auf dem ersten Behälter (8) vorgesehen ist, um eine Verbindung mit dem Fluid herzustellen, das im besagten Behälter (8) eingeschlossen ist.
